(19) **Europäisches Patentamt / European Patent Office / Office européen des brevets**

(11) **EP 4 421 163 A1**

(12) **EUROPEAN PATENT APPLICATION**
published in accordance with Art. 153(4) EPC

(43) Date of publication:
**28.08.2024 Bulletin 2024/35**

(21) Application number: **22883551.8**

(22) Date of filing: **18.10.2022**

(51) International Patent Classification (IPC):
**C12N 5/00** (2006.01)     **C12N 5/071** (2010.01)
**C12Q 1/02** (2006.01)     **G01N 33/15** (2006.01)

(52) Cooperative Patent Classification (CPC):
**C12N 5/00; C12N 5/06; C12Q 1/02; G01N 33/15**

(86) International application number:
**PCT/JP2022/038703**

(87) International publication number:
**WO 2023/068247 (27.04.2023 Gazette 2023/17)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **19.10.2021  JP 2021170989**

(71) Applicants:
• **JSR Corporation**
  **Tokyo 105-8640 (JP)**
• **Kendai Translational Research Center**
  **Takasaki-shi, Gunma 370-0033 (JP)**

(72) Inventors:
• **MASUDA Norio**
  **Tokyo 105-8640 (JP)**
• **OKADA Ryo**
  **Tokyo 105-8640 (JP)**
• **OGIHARA Takuo**
  **Takasaki-shi, Gunma 370-0033 (JP)**

(74) Representative: **TBK**
  **Bavariaring 4-6**
  **80336 München (DE)**

(54) **BIOMIMETIC SYSTEM AND METHOD FOR MANUFACTURING SAME**

(57)     A biomimetic system including: a container; and a human cholangiocyte-like cell-containing membrane, in which the human cholangiocyte-like cell-containing membrane includes a permeable base plate and a two-dimensional tissue of human cholangiocyte-like cells stacked on one surface of the permeable base plate, the human cholangiocyte-like cell-containing membrane divides the container into a first compartment and a second compartment, a one surface side of the permeable base plate is exposed in the first compartment, the other surface side of the permeable base plate is exposed in the second compartment, the human cholangiocyte-like cells express P-gp, and an efflux ratio calculated by Formula (1) is 1.5 or more, efflux ratio = (permeation rate of rhodamine 123 that permeates from the second compartment to the first compartment)/(permeation rate of rhodamine 123 that permeates the second compartment from the first compartment) ... (1)

FIG. 1

EP 4 421 163 A1

**Description**

[Technical Field]

The present invention relates to a biomimetic system and a method for manufacturing the same. Priority is claimed on Japanese Patent Application No. 2021-170989, filed October 19, 2021, the content of which is incorporated herein by reference.

[Background Art]

Since a significant number of drugs are metabolized in liver, injury of the liver exposed to the drugs is a side effect that cannot be avoided. Drug-induced liver injury (DILI) is a major cause of the withdrawal of candidate drugs in the clinical stage and the withdrawal of pharmaceutical products from the market.

MDR1 (multidrug resistance 1) is also referred to as P-glycoprotein (P-gp) and is mainly a transporter that excretes lipid-soluble drugs from the inside to the outside of cells. Whether a drug serves as a substrate of P-gp or whether a drug exhibits an inhibitory action on P-gp is an item that is important for evaluating cholestatic DILI and is also considered important in the guidance of the United States Food and Drug Administration (FDA).

For this reason, it has been attempted to develop a highly safe pharmaceutical product by predicting DILI in the early stage of drug discovery by using a biomimetic system (Micro-physiological system, MPS) using human hepatocytes (see, for example, Non-Patent Document 1 to Non-Patent Document 3).

In order to realize a biomimetic system for predicting DILI, hepatocytes that sufficiently express transporters need to form a membrane that is bound by tight junctions (tight junctions).

However, it is difficult to stably acquire primary human hepatocytes. In addition, there is a problem that the primary human hepatocytes hardly proliferate, and liver functions such as transporters are deteriorated over time.

Therefore, in Non-Patent Document 1, HepG2 cells, which are a cell line derived from human liver cancer, are used in place of the primary human hepatocytes. In addition, in Non-Patent Document 2, human hepatic progenitor cells induced from human iPS cells are used in place of the primary human hepatocytes.

[Citation List]

[Non-Patent Document]

[Non-Patent Document 1]
Chen C., et al., Kanglaite enhances the efficacy of cisplatin in suppression of hepatocellular carcinoma via inhibiting CKLF1 mediated NF-kappaB pathway and regulating transporter mediated drug efflux, Journal of Ethnopharmacology, 264, 113388, 2021.
[Non-Patent Document 2]
Asai A., et al., Paracrine signals regulate human liver organoid maturation from induced pluripotent stem cells, Development, 144, 1056-1064, 2017.
[Non-Patent Document 3]
Takayama K., et al., Prediction of interindividual differences in hepatic functions and drug sensitivity by using human iPS-derived hepatocytes, PNAS, 111 (47), 16772-16777, 2014.

[Summary of Invention]

[Technical Problem]

However, membranes formed from HepG2 cells do not have sufficient expression of transporters and have weak alignment properties. In addition, in order to form a membrane from human hepatic progenitor cells, it is necessary to co-culture the human hepatic progenitor cells with human umbilical vein endothelial cells (HUVEC) or breast cancer cells. For this reason, the human hepatic progenitor cells are significantly different from human hepatocytes, and there is room for improvement as an evaluation tool.

Accordingly, an object of the present invention is to provide a biomimetic system useful for the evaluation of biliary excretion, and a method for manufacturing the biomimetic system.

[Solution to Problem]

The present invention includes the following embodiments.

[1] A biomimetic system including:

a container; and
a human cholangiocyte-like cell-containing membrane,
in which the human cholangiocyte-like cell-containing membrane includes a permeable base plate and a two-dimensional tissue of human cholangiocyte-like cells stacked on one surface of the permeable base plate,
the human cholangiocyte-like cell-containing membrane divides the container into a first compartment and a second compartment,
the one surface side of the permeable base plate is exposed in the first compartment,
the other surface side of the permeable base plate is exposed in the second compartment,
the human cholangiocyte-like cells express P-glycoprotein (P-gp), and
an efflux ratio calculated by Formula (1) is 1.5 or more,

Efflux ratio = (Permeation rate of rhodamine 123 permeating from the second
compartment to the first compartment in a case where rhodamine 123 (CAS number:
62669-70-9) is added to the second compartment)/(permeation rate of rhodamine 123      (1)
permeating from the first compartment to the second compartment in a case where
rhodamine 123 is added to the first compartment)

[2] The biomimetic system according to [1], in which a transepithelial electrical resistance of the two-dimensional tissue is 100 $\Omega \cdot cm^2$ or greater.
[3] The biomimetic system according to [1] or [2], in which the two-dimensional tissue is a single layer of the human cholangiocyte-like cells.
[4] The biomimetic system according to any one of [1] to [3], in which the permeable base plate is a base plate coated with collagen or laminin, or a combination of collagen and laminin.
[5] The biomimetic system according to any one of [1] to [4], in which the human cholangiocyte-like cells further express at least one or more transporters selected from the group consisting of MRP2, BSEP, and BCRP.
[6] A method for manufacturing a biomimetic system, the method including:

a step of seeding human cholangiocyte-like cells on one surface of a permeable base plate in a container divided into a first compartment and a second compartment by the permeable base plate;
a step of culturing the human cholangiocyte-like cells in a culture medium including 0.001 to 5 v/v% of an extracellular matrix so that, consequently, a human cholangiocyte-like cell-containing membrane in which a two-dimensional tissue of the human cholangiocyte-like cells is stacked on the one surface of the permeable base plate is formed.

[7] The method for manufacturing a biomimetic system according to [6], in which the extracellular matrix includes laminin.
[8] A method for manufacturing a biomimetic system, the method including:

a step of seeding human cholangiocyte-like cells on one surface of a permeable base plate in a container divided into a first compartment and a second compartment by the permeable base plate;
a step of culturing the human cholangiocyte-like cells in a culture medium so that, consequently, a human cholangiocyte-like cell-containing membrane in which a two-dimensional tissue of the human cholangiocyte-like cells is stacked on the one surface of the permeable base plate is formed,
in which the permeable base plate is a base plate coated with collagen or laminin, or a combination of collagen and laminin.

[9] The method for manufacturing a biomimetic system according to any one of [6] to [8], in which a seeding density of the human cholangiocyte-like cells is 5,000 to 200,000 cells/cm$^2$.
[10] The method for manufacturing a biomimetic system according to any one of [6] to [9], in which the culture medium contains one kind or two or more kinds of factors selected from the group consisting of a Wnt signaling pathway activator, epidermal growth factor (EGF), a fibroblast growth factor (FGF), hepatocyte growth factor (HGF), and a cAMP signaling pathway activator.

[11] The method for manufacturing a biomimetic system according to any one of [6] to [10], in which the culture medium contains a TGFβ signaling pathway inhibitor.

[12] The method for manufacturing a biomimetic system according to any one of [6] to [11], in which the culture medium substantially does not contain one kind or two or more kinds selected from the group consisting of DAPT, a Wnt signaling pathway inhibitor, and dimethyl sulfoxide (DMSO).

[13] The method for manufacturing a biomimetic system according to any one of [6] to [12], in which the human cholangiocyte-like cells are a human cholangiocyte-like cell line established from human primary hepatocytes.

[14] A biomimetic system obtained by the method for manufacturing a biomimetic system according to any one of [6] to [13].

[15] A method for screening a test substance, the method including:

a step of bringing a test substance into contact with the human cholangiocyte-like cell-containing membrane of the biomimetic system according to any one of [1] to [5] and [14].

[Advantageous Effects of Invention]

According to the present invention, a biomimetic system useful for the evaluation of biliary excretion, and a method for manufacturing the biomimetic system can be provided.

[Brief Description of Drawings]

FIG. 1 is a schematic diagram showing an example of a biomimetic system.

FIG. 2 is a graph showing results of examining expression levels of bile duct marker genes and transporter genes in human cholangiocyte-like cells in Experimental Example 1.

FIG. 3 shows optical microphotographs of human cholangiocyte-like cell-containing membranes of biomimetic systems in Experimental Example 2.

FIG. 4A is a graph showing results of measuring transport amounts of a substrate transported by P-gp in Experimental Example 3.

FIG. 4B is a graph showing results of measuring the transport amounts of the substrate transported by P-gp in Experimental Example 3.

FIG. 5 shows optical microphotographs of human cholangiocyte-like cell-containing membranes of biomimetic systems in Experimental Example 4.

FIG. 6A is a graph showing results of measuring the transport amounts of the substrate transported by P-gp in Experimental Example 4.

FIG. 6B is a graph showing results of measuring the transport amounts of the substrate transported by P-gp in Experimental Example 4.

FIG. 6C is a graph showing results of measuring the transport amounts of the substrate transported by P-gp in Experimental Example 4.

FIG. 6D is a graph showing results of measuring the transport amounts of the substrate transported by P-gp in Experimental Example 4.

FIG. 7A is a graph showing results of measuring the transport amounts of the substrate transported by P-gp in Experimental Example 6.

FIG. 7B is a graph showing results of measuring the transport amounts of the substrate transported by P-gp in Experimental Example 6.

FIG. 7C is a graph showing results of measuring the transport amounts of the substrate transported by P-gp in Experimental Example 6.

FIG. 8 is a graph showing results of examining expression levels of transporter genes in human cholangiocyte-like cells in Experimental Example 7.

FIG. 9 is images showing results of observing the expression of transporters in a human cholangiocyte-like cell-containing membrane of a biomimetic system in Experimental Example 8 with a confocal quantitative image cytometer.

FIG. 10A is a graph showing results of measuring the transport amounts of the substrate transported by MRP2 in Experimental Example 9.

FIG. 10B is a graph showing results of measuring the transport amounts of the substrate transported by MRP2 in Experimental Example 9.

FIG. 11A is a graph showing results of measuring the transport amounts of the substrate transported by MRP2 in Experimental Example 9.

FIG. 11B is a graph showing results of measuring the transport amounts of the substrate transported by MRP2 in Experimental Example 9.

FIG. 12A is a graph showing results of measuring the transport amounts of the substrate transported by BCRP in Experimental Example 10.

FIG. 12B is a graph showing results of measuring the transport amounts of the substrate transported by BCRP in Experimental Example 10.

FIG. 13A is a graph showing results of measuring the transport amounts of the substrate transported by BCRP in Experimental Example 10.

FIG. 13B is a graph showing results of measuring the transport amounts of the substrate transported by BCRP in Experimental Example 10.

[Description of Embodiments]

Hereinafter, embodiments of the present invention will be described in detail, with reference to the drawings in some cases. In the drawings, the same or equivalent portions will be assigned with the same or corresponding reference numerals, and any redundant description will not be repeated. The dimensional ratio in each diagram has some portions exaggerated for explanation and does not necessarily match the actual dimensional ratio.

Unless particularly stated otherwise, each component mentioned as an exemplary example in the present specification, for example, each of the components included in the culture medium and the components used in each step can be used singly or can be used in combination of two or more kinds thereof.

In the present specification, a notation representing a numerical value range such as "1 to 1000" has the same meaning as "1 or more and 1000 or less". In addition, a notation including a plurality of the terms "or more" and "or less" such as "1 to 1000, and preferably 10 to 100" has the same meaning as "1 or more and 1000 or less, 1 or more and 100 or less, 10 or more and 1000 or less, or 10 or more and 100 or less".

In the present specification, the phrase "culture medium including substance X" and "in the presence of substance X" mean a culture medium to which an exogenous substance X has been added, a culture medium including an exogenous substance X, or in the presence of an exogenous substance X. That is, in a case where a cell or tissue present in the culture medium endogenously expresses, secretes, or manufactures the substance X, it should be noted that the endogenous substance X is distinguished from the exogenous substance X, and a culture medium which does not include the exogenous substance X does not fall under the category of "culture medium including substance X" even when the culture medium includes the endogenous substance X.

[Biomimetic system]

In one embodiment, the present invention provides a biomimetic system including: a container; and a human cholangiocyte-like cell-containing membrane, in which the human cholangiocyte-like cell-containing membrane includes a permeable base plate and a two-dimensional tissue of human cholangiocyte-like cells stacked on one surface of the permeable base plate, the human cholangiocyte-like cell-containing membrane divides the container into a first compartment and a second compartment, the one surface side of the permeable base plate is exposed in the first compartment, the other surface side of the permeable base plate is exposed in the second compartment, the human cholangiocyte-like cells express P-glycoprotein (P-gp), and the efflux ratio calculated by Formula (1) is 1.5 or more.

Efflux ratio = (Permeation rate of rhodamine 123 permeating from the second compartment to the first compartment in a case where rhodamine 123 (CAS number: 62669-70-9) is added to the second compartment)/(permeation rate of rhodamine 123 permeating from the first compartment to the second compartment in a case where rhodamine 123 is added to the first compartment)  (1)

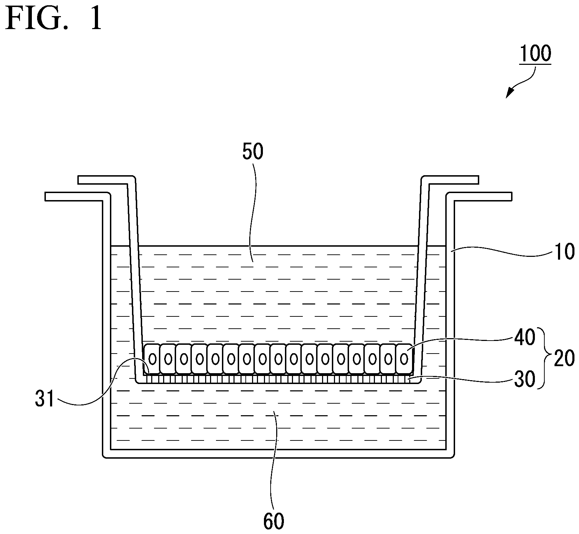

FIG. 1 is a schematic diagram showing an example of a biomimetic system of the present embodiment. As shown in FIG. 1, a biomimetic system 100 includes a container 10 and a human cholangiocyte-like cell-containing membrane 20. In addition, the human cholangiocyte-like cell-containing membrane 20 includes a permeable base plate 30 and a two-dimensional tissue of human cholangiocyte-like cells 40, which is stacked on one surface 31 of the permeable base plate 30. The human cholangiocyte-like cell-containing membrane 20 divides the container 10 into a first compartment 50 and a second compartment 60. Then, the one surface 31 side of the permeable base plate 30 is exposed in the first compartment 50, and the other surface side of the permeable base plate 30 is exposed in the second compartment 60. In addition, the human cholangiocyte-like cells 40 express P-gp, and the efflux ratio calculated by the above-described Formula (1) is 1.5 or more.

In the present specification, a human cholangiocyte-like cell means a cell that is functionally and morphologically equivalent to a human cholangiocyte in vivo and thus can be said in a different way as a human cholangiocyte. The human cholangiocyte-like cell can also be referred to as a cell that expresses a bile duct marker such as keratin 7 (KRT7), keratin 19 (KRT19), SRY-box transcription factor 9 (SOX9), HNF1 homeobox B (NHF1b), CF transmembrane conductance regulator (CFTR), or cytokeratin 19 (CK19). As the human cholangiocyte-like cell, a cell derived from a human bile duct organoid can be suitably used.

A human bile duct organoid can be obtained by subjecting human primary hepatocytes to organoid culture (three-dimensional culture) in a culture medium that does not include interleukin 6 (IL-6). Since the culture medium does not include IL-6, hepatic parenchymal cells among the human primary hepatocytes die during the culture, and only cholangiocytes survive and proliferate. In addition, by performing organoid culture, human cholangiocyte-like cells established as a cell line, which have higher expression of transporters, are stably available, and are capable of proliferating for a long period of time, can be obtained.

As the permeable base plate, a plate-shaped or membrane-shaped support that does not allow cells to permeate therethrough but allows components in the culture medium to permeate therethrough, can be used. It is preferable that the permeable base plate is a base plate showing lower drug adsorption. More specific examples of the permeable base plate include a porous membrane. Examples of the material of the porous membrane include polycarbonate, polyethylene terephthalate, and polytetrafluoroethylene. In addition, the pore size of the porous membrane is preferably about 0.1 μm to 8 μm. The permeable base plate may be disposed on the well bottom surface of a Transwell insert, which is used in combination with a dish or a well plate. In this case, a two-dimensional tissue of human cholangiocyte-like cells is stacked on the well bottom surface of the Transwell insert. It is preferable that the permeable base plate is coated with collagen or laminin, or a combination of collagen and laminin.

Examples of the container include a container in which cells can be cultured. More specific examples of the container include a dish, a well of a well plate, and a fluid device.

For example, in a case where the container is a well of a well plate having a Transwell insert mounted therein, the first compartment is the inside of the Transwell insert. In this case, the second compartment is the inside of the well and the outside of the Transwell insert.

P-gp is a kind of transporter. The NCBI accession number of the amino acid sequence of human P-gp is NP_000918.2, NP_001335873.1, NP_001335874.1, NP_001335875.1, or the like. Rhodamine 123 is one of the substrates for P-gp.

In addition to P-gp, human cholangiocyte-like cells express transporters such as mitochondrial 37S ribosomal protein MRP2 (MRP2), ATP binding cassette subfamily B member 11 (BSEP), and ATP binding cassette subfamily G member 2 (BCRP). The NCBI accession number for the amino acid sequence of human MRP2 is NP_015492.1. The NCBI accession number of the amino acid sequence of human BSEP is NP_003733.2. The NCBI accession number of the amino acid sequence of human BCRP is NP_001244315.1, NP_001335914.1, NP_001335915.1, or the like.

As described above, it is difficult to stably obtain primary human hepatocytes. In contrast, when a human liver organoid which is an established cell line, it is possible to stably obtain human hepatocytes.

However, it has been conventionally difficult to form a membrane joined by tight junctions by using a human liver organoid. The reason for this is that in order to form a membrane having alignment properties and tight junctions using a human liver organoid, it is necessary to use an extracellular matrix; however, when a human liver organoid is embedded in the extracellular matrix, the human liver organoid turns into a cyst form.

In contrast, the human liver organoid can be formed into a single layer membrane by sandwiching the human liver organoid on a membrane of a Transwell insert with an extracellular matrix and culturing the human liver organoid. However, the membrane obtained by this method is even morphologically different from the liver tissue in vivo. In addition, the extracellular matrix remains in the evaluation tool and traps the excretion. For this reason, it is difficult to use the membrane as an evaluation tool.

In the biomimetic system of the present embodiment, a membrane is formed not by using human hepatocytes but by using human cholangiocyte-like cells. The inventors of the present invention have found that a membrane having alignment properties and tight junctions can be formed by culturing human cholangiocyte-like cells in a culture medium including a small amount of an extracellular matrix, thereby completing the present invention. Here, having alignment properties means that the efflux ratio calculated by the above-described Formula (1) is not 1. In the biomimetic system of the present embodiment, the efflux ratio is 1.5 or more, and preferably 2.0 or more. Although not particularly limited, the upper limit of the efflux ratio is approximately 20.

Human cholangiocytes express different types of transporters as compared with human hepatic parenchymal cells. However, in pharmacokinetics research, it is not necessarily essential that all liver functions are maintained to be highly expressed, and even when only some functions are maintained to be expressed, the cells can be used as an evaluation tool for drug discovery research.

The human cholangiocyte-like cell-containing membrane in the biomimetic system of the present embodiment is useful as an evaluation tool for drug discovery research since it expresses P-gp that is involved in biliary excretion.

In the biomimetic system of the present embodiment, it is also important not to differentiate the human cholangiocyte-like cells into hepatic parenchymal cells (hepatocytes). In a case where human cholangiocyte-like cells are differentiated into hepatic parenchymal cells, it tends to be difficult to form a membrane.

The biomimetic system of the present embodiment is a cell-based assay system and can also be said to be a human liver model. The biomimetic system of the present embodiment makes it possible to evaluate drug-induced liver injury (DILI) in vitro.

It is preferable that the transepithelial electrical resistance of the two-dimensional tissue is 100 $\Omega \cdot cm^2$ or greater. When the transepithelial electrical resistance is in the above-described range, it indicates that tight junctions have been sufficiently formed, and evaluation results for transporters which are closer to the evaluation results in the living body can be obtained.

In the biomimetic system of the present embodiment, it is preferable that the two-dimensional tissue is a single layer of the human cholangiocyte-like cells.

The biomimetic system of the present embodiment may contain human hepatocyte-like cells in the second compartment. In the present specification, a human hepatocyte-like cell means a cell that is functionally and morphologically equivalent to a human hepatocyte in vivo and thus can be said in a different way as a human hepatocyte. The human hepatocyte-like cell can also be referred to as a cell that expresses hepatocyte markers such as hepatocyte nuclear factor 4 alpha (HNF4a), alpha fetoprotein (AFP), and albumin (ALB). As the human hepatocyte-like cells, cells derived from a human liver organoid can be suitably used.

[Method for manufacturing biomimetic system]

According to an embodiment, the present invention provides a method for manufacturing a biomimetic system, the method including: a step of seeding human cholangiocyte-like cells on one surface of a permeable base plate in a container divided into a first compartment and a second compartment by the permeable base plate; and a step of culturing the human cholangiocyte-like cells in a culture medium including 0.001 to 5 v/v% of an extracellular matrix so that, consequently, a human cholangiocyte-like cell-containing membrane in which a two-dimensional tissue of the human cholangiocyte-like cells is stacked on the one surface of the permeable base plate is formed. The above-mentioned biomimetic system can be manufactured by the manufacturing method according to the present embodiment.

Alternatively, according to another embodiment, the present invention provides a method for manufacturing a biomimetic system, the method including: a step of seeding human cholangiocyte-like cells on one surface of a permeable base plate in a container divided into a first compartment and a second compartment by the permeable base plate; and a step of culturing the human cholangiocyte-like cells in a culture medium so that, consequently, a human cholangiocyte-like cell-containing membrane in which a two-dimensional tissue of the human cholangiocyte-like cells is stacked on the one surface of the permeable base plate is formed, in which the permeable base plate is a base plate coated with collagen or laminin, or a combination of collagen and laminin. The above-mentioned biomimetic system can also be manufactured by such a manufacturing method.

In the manufacturing method of the present embodiment, the matters concerning the permeable base plate, the first compartment, the second compartment, the container, and the human cholangiocyte-like cell are similar to the matters described above.

In the manufacturing method of the present embodiment, the seeding density of the human cholangiocyte-like cells is usually 5,000 to 200,000 cells/cm$^2$, preferably 10,000 to 150,000 cells/cm$^2$, and more preferably 20,000 to 100,000/cm$^2$. When the seeding density is within the above-described range, a film having a favorable transepithelial electrical resistance of 100 $\Omega \cdot cm^2$ or greater in a short period of time, for example, about 5 to 30 days. In addition, it is preferable that the human cholangiocyte-like cells are seeded in the central part of the permeable base plate because the human cholangiocyte-like cells can be well dispersed.

In the manufacturing method of the present embodiment, the culture medium for culturing the human cholangiocyte-like cells includes a small amount of an extracellular matrix. Here, the term "a small amount" means a concentration to an extent that the extracellular matrix does not gelate, and specifically, the small amount is 0.001 to 5 v/v%, preferably 0.001 to 4 v/v%, and more preferably 0.001 to 3 v/v%, on the basis of the entire amount of the culture medium. It is preferable that both the culture medium used for the first compartment and the culture medium used for the second compartment include an extracellular matrix.

An extracellular matrix is a substance that serves as a scaffold for cells in cell culture. Examples of a commercially available extracellular matrix include Matrigel (registered trademark), human type laminin (Sigma-Aldrich Co., LLC), and product name "Mebiol Gel" (Mebiol, Inc.).

Examples of a component of the extracellular matrix include a component included in the basement membrane, and a glycoprotein present in the intercellular space. Examples of the component included in the basement membrane include type IV collagen, laminin, heparan sulfate proteoglycan, and entactin. Examples of the glycoprotein present in the intercellular space include collagen, laminin, entactin, fibronectin, fibrinogen, and heparin sulfate. Regarding the extracellular matrix, an artificial water-soluble polymer may also be mentioned. Examples of the artificial water-soluble polymer include carboxymethyl cellulose, a polymer containing an azobenzene group and a cyclodextrin group, and calcium alginate. These compounds may be used singly, or two or more kinds thereof may be used in combination. It is preferable that the extracellular matrix includes laminin.

As will be described later in the Examples, the inventors of the present invention found that a two-dimensional tissue, that is, a membrane, can be formed by culturing human cholangiocyte-like cells in a culture medium including a small amount of an extracellular matrix. This human cholangiocyte-like cell-containing membrane had alignment properties and exhibited a transepithelial electrical resistance of 100 $\Omega \cdot cm^2$ or greater.

As the human cholangiocyte-like cell, a cell derived from a human bile duct organoid can be suitably used. The human bile duct organoid can be obtained by subjecting human primary hepatocytes to organoid culture (three-dimensional culture) in a culture medium not containing IL-6. Since the culture medium does not include IL-6, hepatic parenchymal cells among the human primary hepatocytes die during the culture, and only cholangiocytes survive and proliferate. In addition, it is possible to obtain human cholangiocyte-like cells with higher P-gp expression by performing organoid culture.

In the biomimetic system of the present embodiment, it is also important not to differentiate the human cholangiocyte-like cells into hepatic parenchymal cells (hepatocytes). In a case where human cholangiocyte-like cells are differentiated into hepatic parenchymal cells, it tends to be difficult to form a membrane.

It is preferable that the culture medium for culturing the human cholangiocyte-like cells contains one kind or two or more kinds of factors selected from the group consisting of a Wnt signaling pathway activator, epidermal growth factor (EGF), a fibroblast growth factor (FGF), hepatocyte growth factor (HGF), and a cAMP signaling pathway activator.

Wnt signal controls the proliferation and differentiation of cells by regulating the protein level of $\beta$-catenin that functions as a transcription promoting factor. Examples of the Wnt signaling pathway activator include Wnt family, R-spondin family, Norrin, and a glycogen synthase (GSK) inhibitor.

Examples of a member of the Wnt family include Wnt1, Wnt2, Wnt2b, Wnt3, Wnt3a, Wnt4, Wnt5a, Wnt5b, Wnt6, Wnt7a, Wnt7b, Wnt8a, Wnt8b, Wnt9a, Wnt9b, Wnt10a, Wnt10b, Wnt11, and Wnt16, and among them, Wnt3a is preferred. The amino acid sequence information of each of these proteins of the Wnt family can be obtained from the NCBI database.

Afamin is known to contribute to the stabilization and solubilization of the Wnt family. Accordingly, as the Wnt signaling pathway activator, a complex of the Wnt family and afamin is more preferred. Afamin is a glycoprotein belonging to albumin family. Examples of afamin include human afamin (GenBank accession number: AAA21612) and bovine afamin (GenBank accession number: DAA28569).

The Wnt family or the complex of the Wnt family and afamin can be used as an aged culture medium (conditioned medium) including those. The concentration of the Wnt family in the conditioned medium is preferably 18 ng/mL to 900 ng/mL. In a case where a conditioned medium in which the concentration of the Wnt family is in the above-described range is used as the Wnt family, the content proportion of the conditioned medium in the culture medium is usually 1 volume (v/v)% to 50 volume (v/v)%, preferably 10 volume (v/v)% to 30 volume (v/v)%, and more preferably 15 volume (v/v)% to 25 volume (v/v)%, in the total volume of the culture medium.

Examples of a member of the R-spondin family include R-spondin 1, R-spondin 2, R-spondin 3, and R-spondin 4. Among these, R-spondin 1 is preferred. The amino acid sequence information of each protein of the R-spondin family can be obtained from the NCBI database. In a case where the R-spondin family binds to Lgr5 in the cell membrane, the R-spondin family is removed from the cell membrane by autoubiquitination, and as a result, Frezzled that induces activation of the Wnt signaling pathway activates the $\beta$-catenin pathway in the cell membrane.

The R-spondin family can be used as a conditioned medium including this R-spondin family at a concentration of 0.13 $\mu$g/mL to 6.5 $\mu$g/mL. In a case where a conditioned medium in which the concentration of the R-spondin family is in the above-described range is used as the R-spondin family, the content proportion of the conditioned medium in the culture medium is usually 1 volume (v/ v)% to 50 volume (v/v)%, preferably 5 volume (v/v)% to 25 volume (v/v)%, and more preferably 8 volume (v/v)% to 20 volume (v/v)%, in the total volume of the culture medium.

A GSK inhibitor is an inhibitor of glycogen synthase 3$\beta$ (GSK3$\beta$). Since GSK3$\beta$ phosphorylates $\beta$-catenin and promotes a degradation reaction of $\beta$-catenin, GSK3$\beta$ acts as a Wnt agonist.

Examples of the GSK inhibitor include CHIR99021 (CAS Number: 252917-06-9), SB216763 (CAS Number: 280744-09-4), SB415286 (CAS Number: 264218-23-7), CHIR98014 (CAS Number: 252935-94-7), AZD1080 (CAS number: 612487-72-6), and LY2090314 (CAS number: 603288-22-8), and among these, CHIR99021 is preferred.

As the Wnt signaling pathway activator, it is preferable to use the Wnt family and the R-spondin family in combination, it is more preferable to use Wnt3a and R-spondin 1 in combination, and it is even more preferable to use a complex of Wnt3a and afamin in combination with R-spondin 1.

EGF is a growth factor that activates an epidermal growth factor receptor (EGFR or ErbB1). Activated EGFR mainly activates the MAPK signaling pathway and also activates the PI3K signaling pathway and the Jak/stat signaling pathway.

The concentration of EGF included in the culture medium is usually 10 ng/mL to 1,000 ng/mL, preferably 50 ng/mL to 500 ng/mL, and more preferably 80 ng/mL to 200 ng/mL.

FGF is preferably one that can bind to FGF receptor 2 (FGFR2) or FGF receptor 4 (FGFR4), and FGF is preferably FGF2, FGF4, FGF7, or FGF10, and particularly preferably FGF10.

The concentration of FGF included in the culture medium is usually 20 ng/mL to 500 ng/mL, preferably 50 ng/mL to 300 ng/mL, and more preferably 80 ng/mL to 150 ng/mL or less.

HGF is a growth factor that activates a Met receptor, and an activated Met receptor activates the HGF-Met signaling pathway. Activation of the HGF-Met signaling pathway promotes activation of the β-catenin pathway and promotes angiogenesis and metalloprotease production.

The concentration of HGF included in the culture medium is generally 10 ng/mL to 1,000 ng/mL, preferably 50 ng/mL to 500 ng/mL, and more preferably 80 ng/mL to 200 ng/mL.

A cAMP signaling pathway activator is a component capable of increasing the intracellular concentration of adenosine monophosphate (AMP) and consequently reactivating cellular receptors. Examples of the cAMP signaling pathway activator include Forskolin (CAS number: 66575-29-9), dibutyryl cAMP (CAS number: 16980-89-5), cholera toxin (CAS number: 9012-63-9), aminophylline (CAS number: 317-34-0), 2,4-dinitrophenol (CAS number: 51-28-5), norepinephrine (CAS number: 108341-18-0), epinephrine (CAS number: 51-43-4), isoproterenol (CAS number: 7683-59-2), isobutylmethylxanthine (CAS number: 28822-58-4), caffeine (CAS number: 58-08-2), and theophylline (CAS number: 58-55 -9), rolipram (CAS number: 61413-54-5), iloprost (CAS number: 73873-87-7), and prostaglandin E1 (CAS number: 745-65-3). The concentration of the cAMP signaling pathway activator included in the culture medium is usually 0.05 μM to 50 μM, preferably 0.5 μM to 30 μM, and more preferably 1 μM to 15 μM.

In the manufacturing method of the present embodiment, it is preferable that the culture medium contains a TGFβ signaling pathway inhibitor. TGFβ signal contributes to the suppression of cell proliferation, differentiation of cells, induction of apoptosis, and the like. A TGF-β signaling pathway inhibitor downregulates the TGFβ signal. The TGFβ signaling pathway inhibitor may be an inhibitor that inhibits the activation of a type 1 receptor or a type II receptor of serine/threonine kinase type receptor, and examples thereof include a TGFβ signaling pathway associated with the inhibition of phosphorylation of Smad2/3, and a BMP inhibitor associated with the inhibition of phosphorylation of Smad 11519.

Examples of the TGFβ signaling pathway inhibitor associated with the inhibition of phosphorylation of Smad213 include A83-01 (CAS number: 909910-43-6), SB-431542 (CAS number: 301836-41-9), SB-505124 (CAS number: 694433-59-5), SB-525334 (CAS number: 356559-20-1), LY364947 (CAS number: 396129-53-6), SD-208 (CAS number: 627536-09-8), and SJN2511 (CAS number: 446859-33-2), and among them, A83-01 is preferred. The concentration of the TGFβ signaling pathway inhibitor in the culture medium is usually 0.05 μM to 50 μM, preferably 0.5 μM to 30 μM, and more preferably 1 μM to 15 μM.

Examples of the BMP inhibitor associated with the inhibition of phosphorylation of Smad1/5/9 include Noggin, Differential screening-selected gene Aberrative in Neuroblastoma (DAN), and a DAN-like protein. Among these, Noggin is preferred. The concentration of the BMP inhibitor in the culture medium is usually 10 ng/mL to 100 ng/mL, preferably 15 ng/mL to 50 ng/mL, and more preferably 20 ng/mL to 30 ng/mL.

Examples of other components that can be contained in the culture medium include antioxidants such as N-acetylcysteine, α-tocopherol acetate, acetylcysteine, ascorbic acid, ascorbyl palmitate, butylhydroxyanisole, butylated hydroxytoluene, and cysteine; and (Leu15)-gastrin I.

In the manufacturing method of the present embodiment, it is preferable that the culture medium for culturing human cholangiocyte-like cells substantially does not contain one kind or two or more kinds selected from the group consisting of DAPT (CAS number: 208255-80-5), a Wnt signaling pathway inhibitor, and dimethyl sulfoxide (DMSO).

In the present specification, the phrase "substantially does not contain" means that a component is intentionally not added, that the component is allowed to be contained at a level below the detection limit, that the component is allowed to be contained such that the amount of the component is insufficient to exhibit an effect, and the like.

In the manufacturing method of the present embodiment, from the viewpoint of promoting the formation of a human cholangiocyte-like cell-containing membrane, it is important not to differentiate the human cholangiocyte-like cells into hepatic parenchymal cells. DAPT, a Wnt signaling pathway inhibitor, and DMSO are factors that induce the differentiation of human hepatic stem cells or human cholangiocytes into human hepatocytes. As these factors are not contained, the differentiation of human cholangiocyte-like cells into hepatocytes is suppressed, and it is easy to favorably form a human cholangiocyte-like cell-containing membrane.

The culture medium for culturing human cholangiocyte-like cells is usually prepared by adding the above-described factors, a culture medium supplement, and the like to a basal culture medium. In the present specification, the basal culture medium refers to a culture medium that includes amino acids, antioxidants, minerals, and carbon sources such as glucose, and optionally includes serum, fatty acids, proteins such as insulin or transferrin, and the like.

Examples of the basal culture medium include BME culture medium, BGJb culture medium, CMRL 1066 culture medium, Glasgow MEM (GMEM) culture medium, Improved MEM Zinc Option culture medium, IMDM culture medium, Medium 199 culture medium, Eagle MEM culture medium, αMEM culture medium, DMEM culture medium, F-12 culture medium, DMEM/F12 culture medium, IMDM/F12 culture medium, Ham's culture medium, RPMI 1640 culture medium, Neurobasal (registered trademark) culture medium (catalogue number: "21103049", Thermo Fisher Scientific, Inc.), and Fischer's culture medium (all including trade names); and mixed culture media of these.

A culture medium supplement is used to fortify the components of a basal culture medium. Examples of the culture medium supplement include L-glutamic acid, a glutamic acid-containing supplement such as "GlutaMax series" (product name, manufactured by Thermo Fisher Scientific, Inc.), an aqueous solution of amino acids such as "MEM Non-Essential Amino Acids Solution" (product name, manufactured by Thermo Fisher Scientific, Inc.), 2-mercaptoethanol, "KnockOut Serum Replacement" (product name, manufactured by Thermo Fisher Scientific, Inc.), Chemically Defined Lipid Concentrate (product name, manufactured by Thermo Fisher Scientific, Inc.), B-27 Supplement (product name, manufactured by Thermo Fisher Scientific, Inc.) including serum-derived proteins such as insulin and albumin, serum, serum substitutes, biotin, cholesterol, linoleic acid, linolenic acid, progesterone, putrescine, retinol, retinyl acetate, sodium selenite, triiodothyronine (T3), DL-α-tocopherol (vitamin E), albumin, insulin, and transferrin; and nerve supplements such as N2 Supplement including human transferrin, bovine insulin, progesterone, putrescine, and sodium selenite.

In the manufacturing method of the present embodiment, it is preferable that the human cholangiocyte-like cells are a human cholangiocyte-like cell line established from human primary hepatocytes.

Here, the human primary hepatocytes may be frozen cells, and the human cholangiocyte-like cell line (human bile duct organoid) may be a cell line obtained by causing cryopreserved cells to sleep. Since the human cholangiocyte-like cell line can be cryopreserved, it is not necessary to maintain the cell line in the form of a biomimetic system for a long period of time, and the human cholangiocyte-like cell line can be caused to sleep and then can be manufactured into a biomimetic system and used.

[Biomimetic system]

In one embodiment, the present invention provides a biomimetic system obtained by the manufacturing method described above.

A human cholangiocyte-like cell-containing membrane in the biomimetic system of the present embodiment expresses P-gp that is involved in bile metabolism. In addition, the human cholangiocyte-like cell-containing membrane exhibits an efflux ratio of 1.5 or more, preferably 2.0 or more, and has alignment properties. In addition, the transepithelial electrical resistance is 100 $\Omega \cdot cm^2$ or greater. Accordingly, the human cholangiocyte-like cell-containing membrane is useful for the evaluation of biliary excretion.

[Method for screening test substance]

In one embodiment, the present invention provides a method for screening a test substance, the method including a step of bringing a test substance into contact with the human cholangiocyte-like cell-containing membrane of the above-mentioned biomimetic system.

According to the screening method of the present embodiment, for example, whether a test substance can serve as a substrate for P-gp, or whether the test substance can exhibit an inhibitory action against P-gp, can be evaluated. As a result, in the early stage of drug discovery, test substances for which it is difficult to induce drug-induced liver injury (DILI) can be accurately screened in vitro. For this reason, the withdrawal of candidate drugs in the clinical stage and the withdrawal of pharmaceutical products from the market can be prevented, and drug discovery can be efficiently promoted.

The screening method of the present embodiment can also be referred to as a method of predicting drug-induced liver injury.

[Examples]

Hereinafter, the present embodiment will be described more specifically based on Examples; however, the present embodiment is not intended to be limited to these Examples. In the following Experimental Examples, devices that had been subjected to sterilization treatment were used in all of the experiments.

[Experimental Example 1]

(Preparation of human cholangiocyte-like cell population)

Human primary frozen hepatocytes (manufactured by BIOPREDIC, HEP187-S) were thawed in a water bath at 37°C and suspended in a 50-mL tube into which a serum-free culture medium (culture medium obtained by adding HEPES, GLUTAMAX, and PENICILIN/STEREPTOMYCIN to Advanced DMEM/F12) had been introduced, followed by centrifugation. After the centrifugation, the supernatant was removed, and precipitated cells were suspended in the above-described serum-free medium to prepare a hepatocyte suspension.

20,000 cells were taken from the above-described hepatocyte suspension and mixed with 50 $\mu$L of Matrigel (manufactured by BD Biosciences, Inc.), and the cells were seeded in a 24-well tissue culture plate and incubated at 37°C for 10 minutes until Matrigel was completely polymerized.

Subsequently, 500 $\mu$L/well of a culture medium to which the components shown in the following Table 1 were added, was overlaid on a basal culture medium (obtained by adding HEPES, GLUTAMAX, and PENICILIN/STEREPTOMYCIN to Advanced DMEM/F12) and cultured at 37°C in the presence of 5% by volume of $CO_2$ to obtain human cholangiocyte-like cells (bile duct organoid).

In Table 1, R-spondin 1, which is a Wnt agonist, was used in the form of a conditioned medium including R-spondin 1, and the concentration of R-spondin 1 in the conditioned medium was 1.3 $\mu$g/mL. Furthermore, Wnt3a, which is a Wnt agonist, was also used in the form of a conditioned medium including a complex of Wnt3a and afamin, as is the case of R-spondin 1, and the concentration of Wnt3a in the conditioned medium was 360 ng/mL.

[Table 1]

| Component | Concentration |
| --- | --- |
| R-spondin 1 | 10% (v/v) |
| Wnt3a | 20% (v/v) |
| Noggin | 25 ng/mL |
| EGF | 50 ng/mL |
| FGF10 | 100 ng/mL |
| HGF | 25 ng/mL |
| Y-27632 | 10 $\mu$M |
| Forskolin | 10 $\mu$M |
| A83-01 | 5 $\mu$M |

(Evaluation of human cholangiocyte-like cells)

After two weeks from the start of culture, total ribonucleic acid (RNA) was extracted from the human cholangiocyte-like cells by using a commercially available kit (trade name: "FastLane Cell cDNA Kit," QIAGEN N.V.), and RNA-seq analysis was performed.

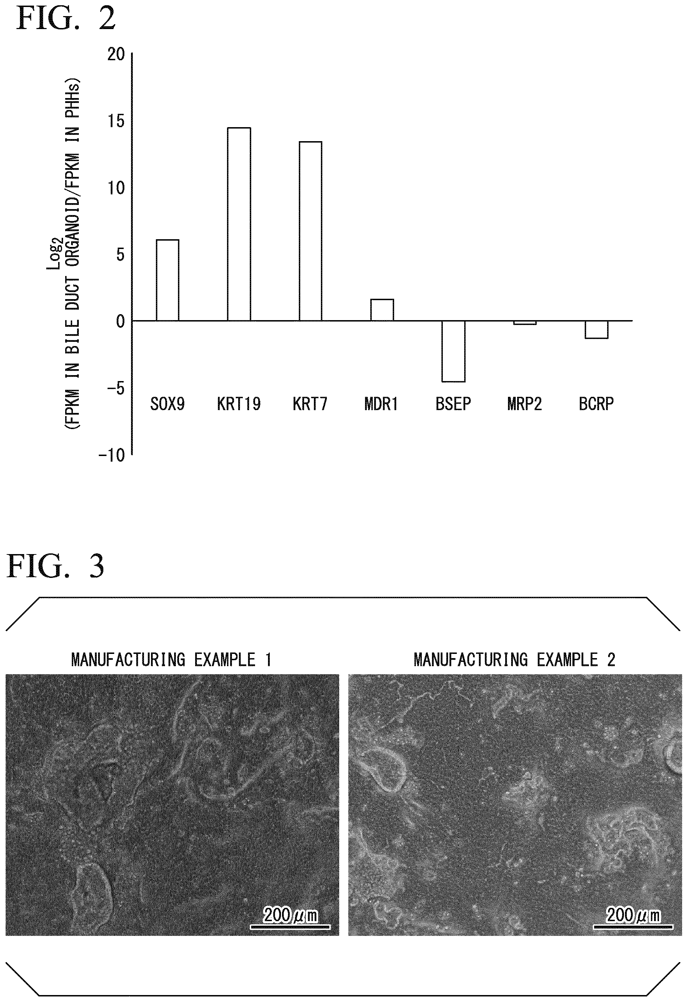

FIG. 2 shows results of calculating expression levels of bile duct marker genes and transporter genes in human cholangiocyte-like cells based on the results of RNA-seq analysis. As the bile duct marker genes, SOX9 gene, KRT19 gene, and KRT7 gene were examined. As the transporter genes, MDR1 (P-gp) gene, BSEP gene, MRP2 gene, and BCRP gene were examined.

In FIG. 2, the axis of ordinate represents the value of $\log_2$ of a ratio between the expression level (Fragments Per Kilobase of exon per Million mapped reads, FPKM) of each gene in the human cholangiocyte-like cells (bile duct organoid) and the expression level (FPKM) of each gene in PHHs (human primary hepatocytes (BIOPREDIC, HEP187-S)).

[Experimental Example 2]

(Manufacture of biomimetic system)

Two types of culture media were each used to manufacture a biomimetic system of Manufacturing Example 1 or Manufacturing Example 2. The culture media used were culture media obtained by adding the components shown in the Table 1 above to each of the basal culture media shown in the following Table 2 and further mixing in 1 v/v% Matrigel (registered trademark) thereto.

The human cholangiocyte-like cells (bile duct organoid) in the Matrigel (registered trademark) of Experimental Example 1 were taken out by enzymatic dissociation, and 30,000 cells were mixed with each of the culture media (500 $\mu$L) to prepare a suspension. 500 $\mu$L of the suspension was placed inside the insert of an insert for cell culture (product name: "ad-MED Vitrigel", 12-well) manufactured by Kanto Chemical Co., Inc., having a structure similar to that of the container of the biomimetic system shown in FIG. 1, each culture medium (1500 μL) was put outside the insert, the cells were cultured at 37°C in the presence of 5% by volume of $CO_2$, and each of the biomimetic systems of Manufacturing Example 1 and Manufacturing Example 2 was obtained. On the bottom surface of the insert, a Vitrigel (registered trademark) membrane in which collagen fibers were densely piled up, was disposed.

FIG. 3 shows optical microphotographs of the human cholangiocyte-like cell-containing membranes of the biomimetic systems of Manufacturing Example 1 and Manufacturing Example 2 on Day 9 from the start of culture.

(Measurement of transepithelial electrical resistance (TEER))

On Day 8, Day 9, and Day 12 from the start of culture, the transepithelial electrical resistance of the human cholangiocyte-like cell-containing membranes of the biomimetic systems of Manufacturing Example 1 and Manufacturing Example 2 was measured by using a commercially available transepithelial electrical resistance measuring apparatus (product name "Millicell ERS-2 Resistance Value Measuring System", Millipore Corporation).

In each of wells for blank measurement in which cells were not seeded, and wells in which cells were seeded, electrodes were arranged to be in contact with the inside of the insert and the outside of the insert, and the electrical resistance (Ω) was measured. A value obtained by subtracting the measurement value (Ω) of the blank from the measurement value (Ω) of a sample and multiplying the resultant value by the membrane area ($cm^2$) (1.0 $cm^2$ in a case of a 12-well plate), was taken as the transepithelial electrical resistance (TEER) ($\Omega \cdot cm^2$). The values of the measured TEER are presented in the following Table 2.

[Table 2]

| | Basal culture medium | TEER ($\Omega \cdot cm^2$) | | |
| --- | --- | --- | --- | --- |
| | | Day 8 | Day 9 | Day 12 |
| Production Example 1 | Advanced DMEM | 267 | 202 | - |
| Production Example 2 | William's E Medium | 110 | 95 | 169 |

As a result, it was revealed that a human cholangiocyte-like cell-containing membrane showing a high TEER value regardless of which culture medium was used, was obtained.

[Experimental Example 3]

(Measurement of efflux ratio by P-gp)

The efflux ratio by P-gp was measured by using the biomimetic system of Manufacturing Example 2 on Day 17 and Day 23 from the start of culture.

Rhodamine 123 (CAS Number: 62669-70-9) (10 μM), which is known to be a substrate for P-gp, was added to the culture medium inside the insert (hereinafter, may be referred to as "side A"), 50 μL of the culture medium on side B was sampled over time, and the transport amount of rhodamine 123 from the inside of the insert to the outside of the insert (hereinafter, may be referred to as "side B") (AtoB) was measured. In addition, rhodamine 123 (10 μM) was added to the culture medium on the side B, 50 μL of the culture medium on the side A was sampled over time, and the transport amount of rhodamine 123 from the side B to the side A (BtoA) was measured.

The transport amounts of rhodamine 123 were measured by measuring the fluorescence intensity of rhodamine 123 with a fluorescence plate reader. Sampling of the culture medium was carried out 15, 30, 45, and 60 minutes after the addition of rhodamine 123 to the culture medium.

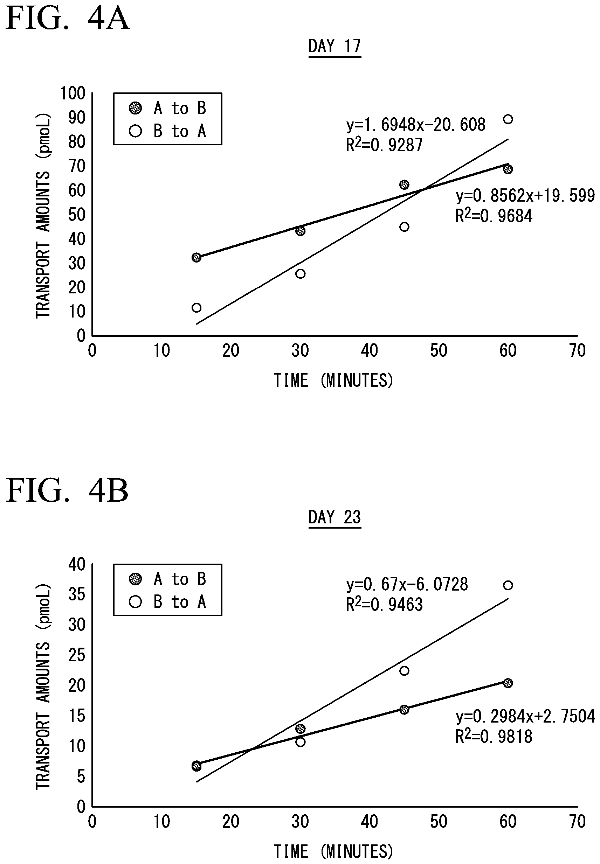

FIG. 4A and FIG. 4B are graphs showing the measurement results of the transport amount (AtoB) and the transport amount (BtoA). FIG. 4A shows the measurement results on Day 17 from the start of culture, and FIG. 4B shows the measurement results on Day 23 from the start of culture. In FIG. 4A and FIG. 4B, the axis of abscissa in the graph represents the time (minutes), and the axis of ordinate represents the amount of transported rhodamine 123 (pmoL). In addition, as shown in FIG. 4A and FIG. 4B, approximation curves were each created based on the graphs of the transport amount (AtoB) and the transport amount (BtoA), and the gradient (permeation rate) was calculated. Subsequently, the efflux ratio (Efflux Ratio (ER)) was calculated based on Formula (2). The results are shown in the following Table 3.

$$\text{Efflux ratio} = (\text{Permeation rate of BtoA})/(\text{permeation rate of AtoB}) \dots (2)$$

[Table 3]

|  | TEER ($\Omega \cdot cm^2$) | Efflux ratio |
|---|---|---|
| Day 17 | 132 | 7.3 |
| Day 23 | 332 | 11.6 |

As a result, it was verified that the human cholangiocyte-like cell-containing membrane of the biomimetic system of Manufacturing Example 2 showed a sufficient efflux ratio and had alignment properties for P-gp.

[Experimental Example 4]

(Examination of influence of concentration of extracellular matrix at time of creating biomimetic system)

<<Preparation of human cholangiocyte-like cell population>>

Cholangiocyte-like cells were obtained by a method similar to that used in Experimental Example 1, except that a culture medium obtained by adding the components shown in the following Table 4 to a basal culture medium (obtained by adding HEPES, GLUTAMAX, PENICILIN/STEREPTOMYCIN, B27 Supplement, and N2 supplement to Advanced DMEM/F12) was used.

[Table 4]

| Component | Concentration |
|---|---|
| R-spondin 1 | 10% (v/v) |
| Wnt3a | 20% (v/v) |
| Noggin | 25 ng/mL |
| EGF | 50 ng/mL |
| FGF10 | 100 ng/mL |
| FGF2 | 100 ng/mL |
| HGF | 25 ng/mL |
| Y-27632 | 10 $\mu$M |
| Forskolin | 10 $\mu$M |
| A83-01 | 5 $\mu$M |
| IGF1 | 50 ng/mL |
| N-Acetyl-cysteine | 1 mM |
| (Leu15)-gastrin I | 10 nM |

<<Manufacture of biomimetic system>>

Biomimetic systems of Manufacturing Example 3 to Manufacturing Example 8 were each manufactured by using culture media having different concentrations of Matrigel (registered trademark). The culture media used were culture media obtained by adding the components shown in the above-described Table 4 to a basal culture medium (William's E Medium) and further mixing in Matrigel (registered trademark) at the concentrations shown in the following Table 5.

The cholangiocyte-like cells in Matrigel (registered trademark) were extracted by enzymatic dissociation, and 30,000 cells were mixed with each culture medium (500 $\mu$L) to prepare a suspension. 200 $\mu$L of the suspension was placed inside the insert of an insert for cell culture (product name: "ad-MED Vitrigel", 24-well) manufactured by Kanto Chemical Co., Inc., having a structure similar to that of the container of the biomimetic system shown in FIG. 1, each culture medium (500 $\mu$L) was put outside the insert, the cells were cultured at 37°C in the presence of 5% by volume of $CO_2$, and each of the biomimetic systems of Manufacturing Example 3 to Manufacturing Example 8 was obtained.

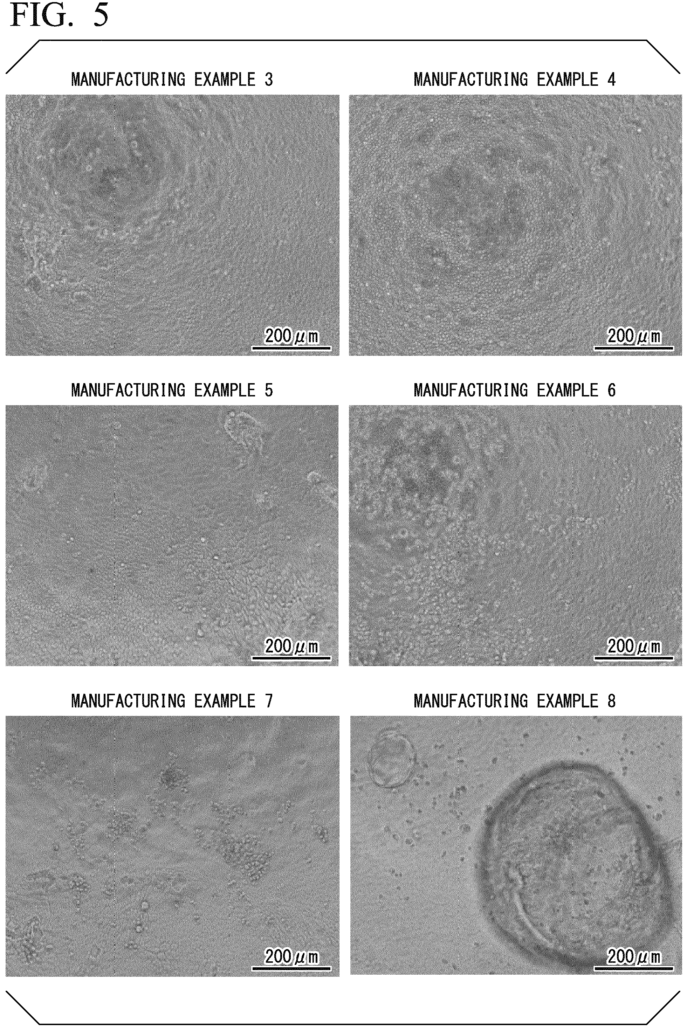

FIG. 5 shows optical photomicrographs of human cholangiocyte-like cell-containing membranes of the biomimetic systems of Manufacturing Example 3 to Manufacturing Example 8 on Day 17 from the start of culture.

<<Measurement of Transepithelial Electrical Resistance (TEER)>>

On Day 11, Day 14, and Day 17 from the start of culture, the transepithelial electrical resistance of the human cholangiocyte-like cell-containing membranes of the biomimetic systems of Manufacturing Example 3 to Manufacturing Example 8 was measured by using a commercially available transepithelial electrical resistance measuring apparatus (product name "Millicell ERS-2 Resistance Value Measuring System", Millipore Corporation).

In each of wells for blank measurement in which cells were not seeded, and wells in which cells were seeded, electrodes were arranged to be in contact with the inside of the insert and the outside of the insert, and the electrical resistance ($\Omega$) was measured. A value obtained by subtracting the measurement value ($\Omega$) of the blank from the measurement value ($\Omega$) of a sample and multiplying the resultant value by the membrane area ($cm^2$) (0.33 $cm^2$ in a case of a 24-well plate), was taken as the transepithelial electrical resistance (TEER) ($\Omega \cdot cm^2$). The values of the measured TEER are presented in the following Table 5.

<<Measurement of efflux ratio by P-gp>>

The efflux ratio by P-gp was measured in the same manner as in Experimental Example 3, by using the biomimetic systems of Manufacturing Examples 3 to 5 and Manufacturing Example 7 on Day 17 from the start of culture.

Rhodamine 123 (10 $\mu$M) was added to the culture medium on the side A, 100 $\mu$L of the culture medium on the side B was sampled over time, 100 $\mu$L of the culture medium was added after sampling, and the transport amount of rhodamine 123 from the side A to the side B (AtoB) was measured. In addition, rhodamine 123 (10 $\mu$M) was added to the culture medium on the side B, 50 $\mu$L of the culture medium on the side A was sampled over time, 50 $\mu$L of the culture medium was added after sampling, and the transport amount of rhodamine 123 from the side B to the side A (BtoA) was measured.

The transport amounts of rhodamine 123 were measured by measuring the fluorescence intensity of rhodamine 123 with a fluorescence plate reader. Sampling of the culture medium was carried out 15, 30, 45, and 60 minutes after the addition of rhodamine 123 to the culture medium.

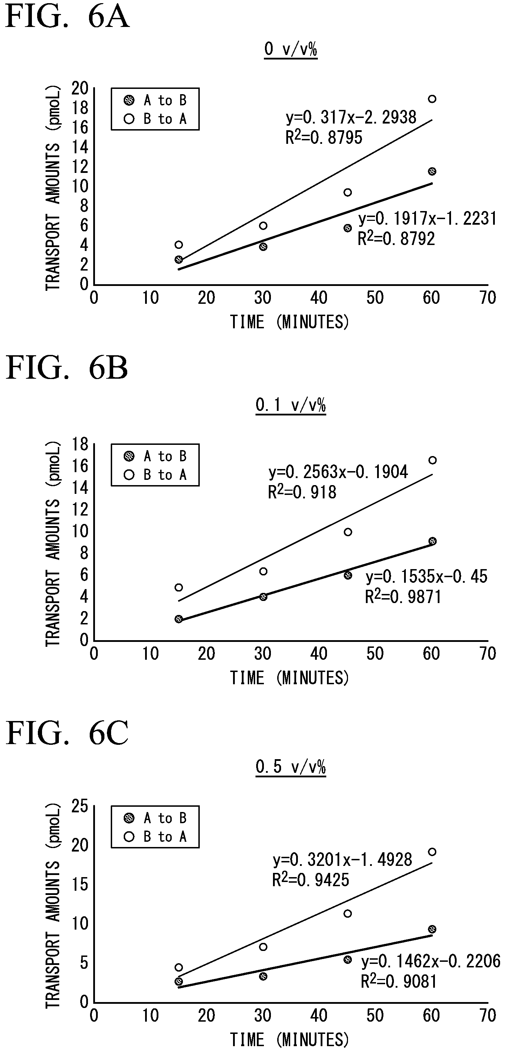

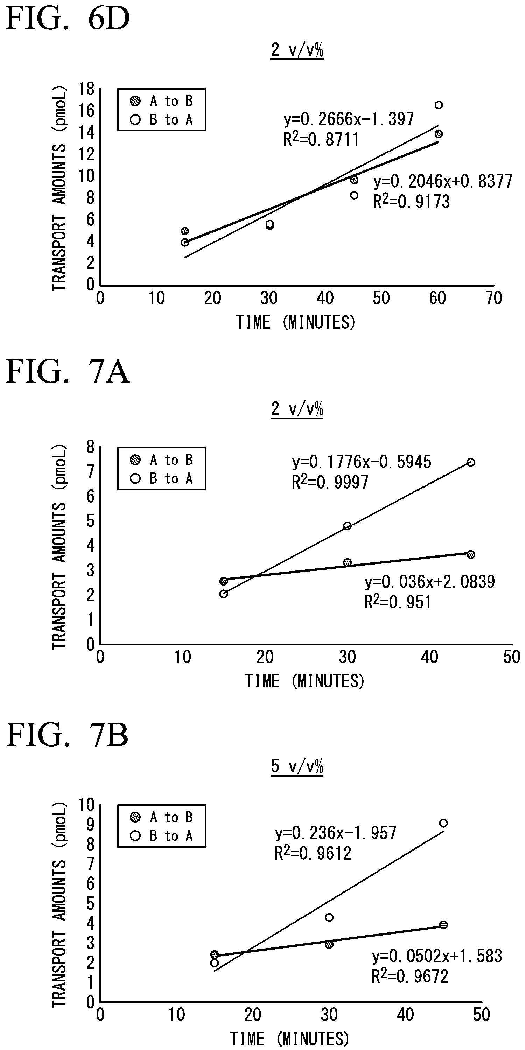

FIG. 6A to FIG. 6D are graphs showing the measurement results of the transport amount (AtoB) and the transport amount (BtoA). The axis of abscissa in the graph represents the time (minutes), and the axis of ordinate represents the amount of transported rhodamine 123 (pmoL). Each of FIG. 6A, FIG. 6B, FIG. 6C, and FIG. 6D presents the results of using a culture medium including Matrigel (registered trademark) at a concentration of 0, 0.1, 0.5, of 2 v/v%. In addition, as shown in FIG. 6A to FIG. 6D, approximation curves were each created based on the graphs of the transport amount (AtoB) and the transport amount (BtoA), and the gradient (permeation rate) was calculated. Subsequently, the efflux ratio was calculated based on the above-described Formula (2). The results are shown in the following Table 5.

[Table 5]

| | Matrigel concentration (v/v%) | TEER ($\Omega \cdot cm^2$), n = 2 | | | Efflux ratio |
| --- | --- | --- | --- | --- | --- |
| | | Day 11 | Day 14 | Day 17 | |
| Production Example 3 | 0 | 7 | 107 | 233 | 1.7 |
| Production Example 4 | 0.1 | 16 | 391 | 208 | 1.7 |
| Production Example 5 | 0.5 | 20 | 543 | 269 | 2.2 |
| Production Example 6 | 1 | 10 | 179 | 255 | - |
| Production Example 7 | 2 | 9 | 89 | 180 | 1.3 |
| Production Example 8 | 10 | 9 | 15 | 31 | - |

From the results of Manufacturing Example 3 to Manufacturing Example 8, it was revealed that it is more preferable when the concentration of the extracellular matrix at the time of manufacturing the biomimetic system is lower.

[Experimental Example 5]

(Verification of effect of P-gp inhibitor)

The effect of verapamil hydrochloride, which is a P-gp inhibitor, was checked. An examination was conducted by using the biomimetic system of Manufacturing Example 5 on Day 17 from the start of culture in Experimental Example 4. Rhodamine 123 (10 $\mu$M) and verapamil hydrochloride (100 $\mu$M) were added to the culture medium on the side A, instead of adding rhodamine 123 (10 $\mu$M). Subsequently, 100 $\mu$L of the culture medium on the side B was sampled over time, 100 $\mu$L of the culture medium was added after the sampling, and the transport amount of rhodamine 123 from the side A to the side B (AtoB) was measured. In addition, instead of adding rhodamine 123 (10 $\mu$M) to the culture medium on the side B, rhodamine 123 (10 $\mu$M) and verapamil hydrochloride (100 $\mu$M) were added thereto. Subsequently, 50 $\mu$L of the culture medium on the side A was sampled over time, 50 $\mu$L of the culture medium was added after the sampling, and the transport amount of rhodamine 123 from the side B to the side A (BtoA) was measured. Subsequently, the efflux ratio was calculated in the same manner as in Experimental Example 4. As a result, the efflux ratio was 1.23.

[Experimental Example 6]

(Biomimetic system having insert coated with Matrigel)

<<Preparation of container having insert coated with Matrigel>>

A Matrigel (registered trademark) solution was prepared with cooled PBS so as to obtain a concentration of 2 v/v%, 5 v/v%, or 10 v/v%. Subsequently, 200 $\mu$L/well each of the Matrigel (registered trademark) solution was placed inside the insert of an insert for cell culture (product name: "ad-MED Vitrigel", 24-well) manufactured by Kanto Chemical Co., Inc., and the cells were incubated for 1 hour in a constant-temperature bath at 37°C. Subsequently, the residual liquid in the wells was removed, and three types of containers having an insert coated with Matrigel (registered trademark) (a 2 v/v% Matrigel-coated container, a 5 v/v% Matrigel-coated container, and a 10 v/v% Matrigel-coated container) were obtained.

<<Manufacture of biomimetic system of Manufacturing Example 9 to Manufacturing Example 11>>

In the same manner as in Experimental Example 4, the cholangiocyte-like cells in the Matrigel (registered trademark) were taken out by enzymatic dissociation, 20,000 cells each were mixed into a culture medium (500 $\mu$L, a culture medium obtained by adding the components shown in the Table 4 to a basal culture medium (William's E Medium)), and a suspension was obtained. Subsequently, 200 $\mu$L of the suspension was placed inside the insert of the above-mentioned Matrigel-coated container. Subsequently, the culture medium (500 $\mu$L) was placed outside the insert, culturing was performed at 37°C in the presence of 5% by volume of $CO_2$ until the transepithelial electrical resistance (TEER) exceeded 100 $\Omega \cdot cm^2$, and the biomimetic systems of Manufacturing Example 9 to Manufacturing Example 11 were manufactured. The culture medium inside the insert and the culture medium outside the insert were exchanged every 4 days.

<<Measurement of efflux ratio by P-gp>>

The efflux ratio by P-gp was measured in the same manner as in Experimental Example 3 by using the biomimetic systems of Manufacturing Example 9 to Manufacturing Example 11.
Rhodamine 123 (10 $\mu$M) was added to the culture medium on the side A, 100 $\mu$L of the culture medium on the side B was sampled over time, 100 $\mu$L of the culture medium was added after sampling, and the transport amount of rhodamine 123 from the side A to the side B (AtoB) was measured. In addition, rhodamine 123 (10 $\mu$M) was added to the culture medium on the side B, 50 $\mu$L of the culture medium on the side A was sampled over time, 50 $\mu$L of the culture medium was added after sampling, and the transport amount of rhodamine 123 from the side B to the side A (BtoA) was measured.
The transport amounts of rhodamine 123 were measured by measuring the fluorescence intensity of rhodamine 123 with a fluorescence plate reader. Sampling of the culture medium was carried out 15, 30, and 45 minutes after the addition of rhodamine 123 to the culture medium.

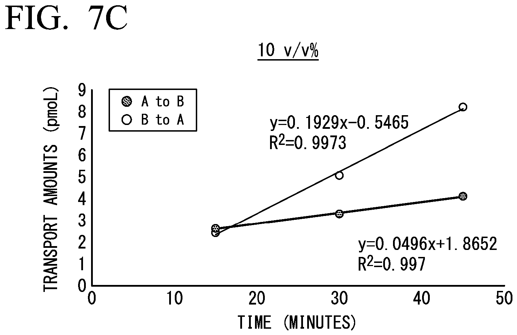

FIG. 7A to FIG. 7C are graphs showing the measurement results of the transport amount (AtoB) and the transport amount (BtoA). Each of FIG. 7A, FIG. 7B, and FIG. 7C presents the results of using a container coated with Matrigel (registered trademark) at a concentration of 2, 5, or 10 v/v%. In addition, as shown in FIG. 7A to FIG. 7C, approximation curves were each created based on the graphs of the transport amount (AtoB) and the transport amount (BtoA), and the gradient (permeation rate) was calculated. Subsequently, the efflux ratio was calculated based on the above-described Formula (2). The results are shown in the following Table 6.

[Table 6]

| | Matrigel concentration (v/v%) | Number of culture days (days) | TEER ($\Omega \cdot cm^2$) | Efflux ratio |
|---|---|---|---|---|
| Production Example 9 | 2 | 14 | 133 | 4.93 |
| Production Example 10 | 5 | 14 | 146 | 4.70 |
| Production Example 11 | 10 | 14 | 146 | 3.89 |

[Experimental Example 7]

(Evaluation of transporters of human cholangiocyte-like cells)

Human cholangiocyte-like cells were obtained by a method similar to that used in Experimental Example 1, total ribonucleic acid (RNA) was extracted from the human cholangiocyte-like cells, and RNA-seq analysis was performed.

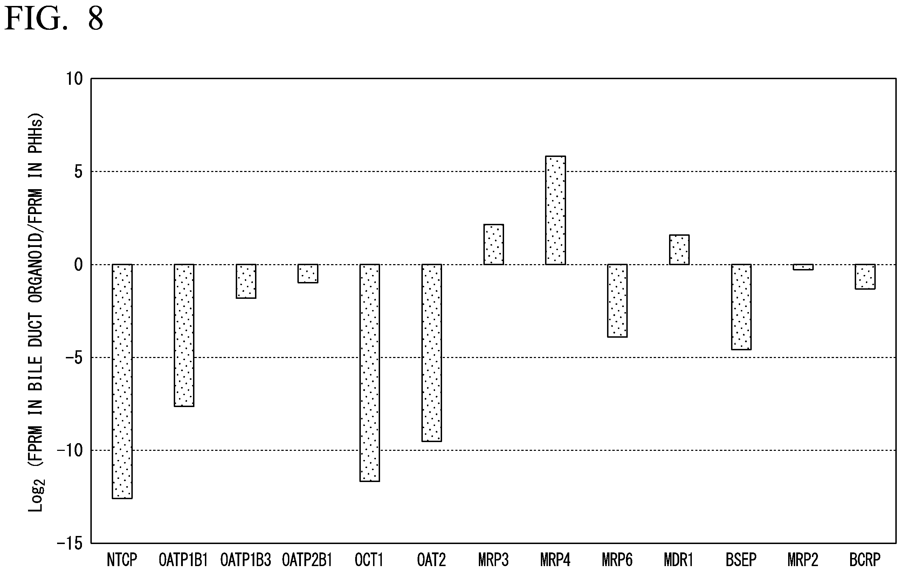

Similarly to FIG. 2, FIG. 8 shows the results of calculating the expression levels of transporter genes related to the uptake of bile into cells in the human cholangiocyte-like cells, and transporter genes related to the excretion of bile from cells in the human cholangiocyte-like cells, based on the results of RNA-seq analysis. Regarding the transporter genes related to bile uptake, NTCP gene, OATP1B1 gene, OATP1B3 gene, OATP2B1 gene, OCT1 gene, OAT2 gene, MRP3 gene, and MRP4 gene were examined, and regarding the transporter genes related to biliary excretion, MRP6 gene, MDR1 gene, BSEP gene, MRP2 gene, and BCRP gene were examined. In FIG. 8, the axis of ordinate has the same meaning as the axis of ordinate in FIG. 2.

[Experimental Example 8]

(Expression of transporters in human cholangiocyte-like cell-containing membrane of biomimetic system)

The expression of transporters in a human cholangiocyte-like cell-containing membrane of a biomimetic system was examined by immunostaining. Human cholangiocyte-like cells were manufactured by a method similar to that used in Experimental Example 1. In addition, a biomimetic system was manufactured in the same manner as in Manufacturing Example 1 of Experimental Example 2, except that the number of culture days was set to 15 days.

An antibody (primary antibody) solution of CK19 (bile duct marker), MDR1, MRP2, and BCRP was placed on the side A of the biomimetic system and then was stained with Alexa Fluor 568 (product of Thermo Fisher Scientific, Inc.)-labeled secondary antibody, and the expression of the transporters in the depth direction of the human cholangiocyte-like cell-containing membrane was observed with a confocal quantitative image cytometer (product name: "CellVoyager CQ1", manufactured by Yokogawa Electric Corporation).

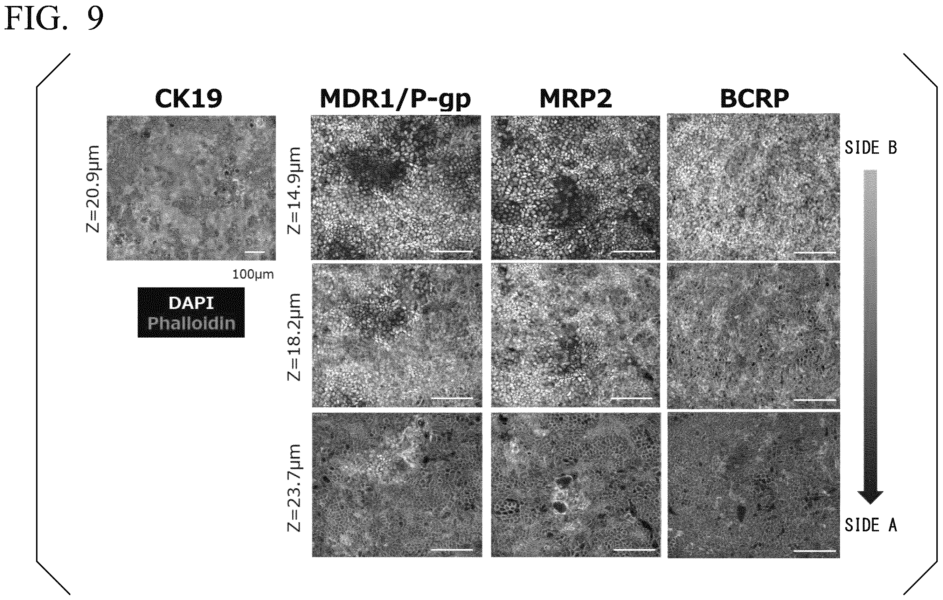

FIG. 9 is an image showing the results of staining CK19, MDR1, MRP2, and BCRP. In addition, the nuclei were stained with DAPI, and the cytoskeleton was stained with Alexa Fluor 488 phalloidin (product of Thermo Fisher Scientific, Inc.). In FIG. 9, Z = 23.7 $\mu$m is the vicinity of the surface of the human cholangiocyte-like cell-containing membrane exposed on the side A, and Z = 14.9 $\mu$m is the vicinity of the surface of the human cholangiocyte-like cell-containing membrane exposed on the side B.

[Experimental Example 9]

(Evaluation of MRP2)

<<Measurement of efflux ratio by CDCFDA>>

The biomimetic system was used to measure the efflux ratio of CDCFDA (CAS number: 127770-45-0), which is a substrate for MRP2.

Human cholangiocyte-like cells were manufactured by a method similar to that used in Experimental Example 1, except that for the components shown in the above Table 1, a culture medium to which PG-002 (product name of PeptiGrowth, Inc. 50 nM) was added was used in place of A83-01 (5 $\mu$M). In addition, a biomimetic system was manufactured in the same manner as in Manufacturing Example 1 of Experimental Example 2, except that the number of culture days was set to 15 days. The TEER of the manufactured biomimetic system was measured by the same method as that used in Experimental Example 2, and the value was 247.34 $\Omega \cdot cm^2$.

The transport amounts of CDCFDA were measured in the same manner as in Experimental Example 3, except that CDCFDA was used in place of rhodamine 123 as a substrate. Regarding CDCFDA, a solution obtained by dissolving CDCFDA in DMSO to a concentration of 1% by mass was used.

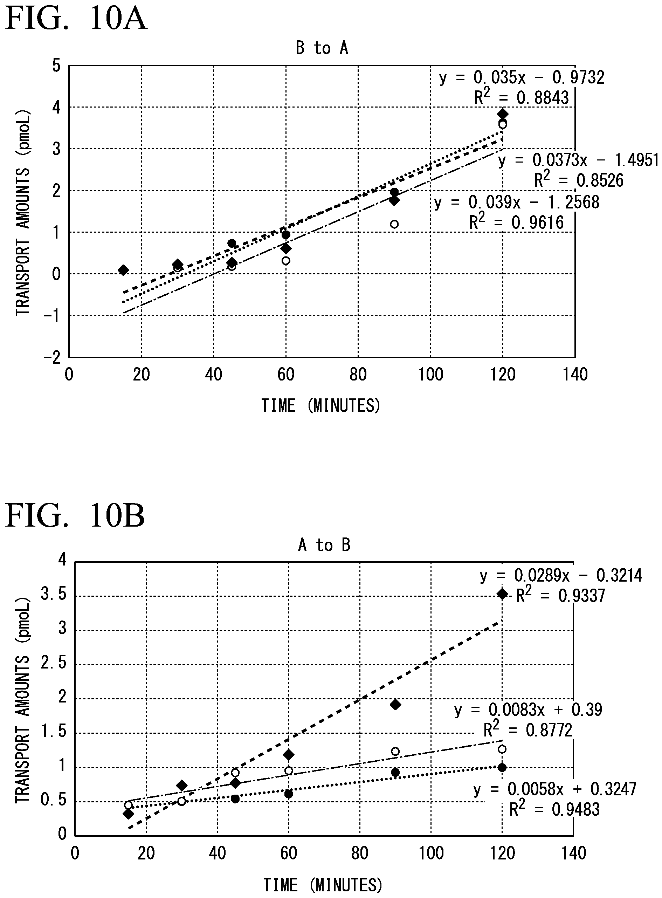

FIG. 10A is a graph showing the results of measuring the transport amount (BtoA) three times, and FIG. 10B is a graph showing the results of measuring the transport amount (AtoB) three times. The axis of abscissa in the graph represents the time (minutes), and the axis of ordinate represents the amount (pmoL) of transported CDCFDA. The efflux ratio calculated in the same manner as in Experimental Example 3 from the average value of the results of the three measurements was about 2.6.

«Verification of effect of MRP2 inhibitor»

The effect of MK571 (CAS Number: 115104-28-4), which is an MRP2 inhibitor, was checked.

The transport amounts of CDCFDA were measured in the same manner as in Experimental Example 5, except that CDCFDA was used in place of rhodamine 123, and furthermore, MK571 was used in place of verapamil hydrochloride. Regarding CDCFDA and MK571, a solution obtained by dissolving each of them in DMSO to a concentration of 0.1% by mass was used.

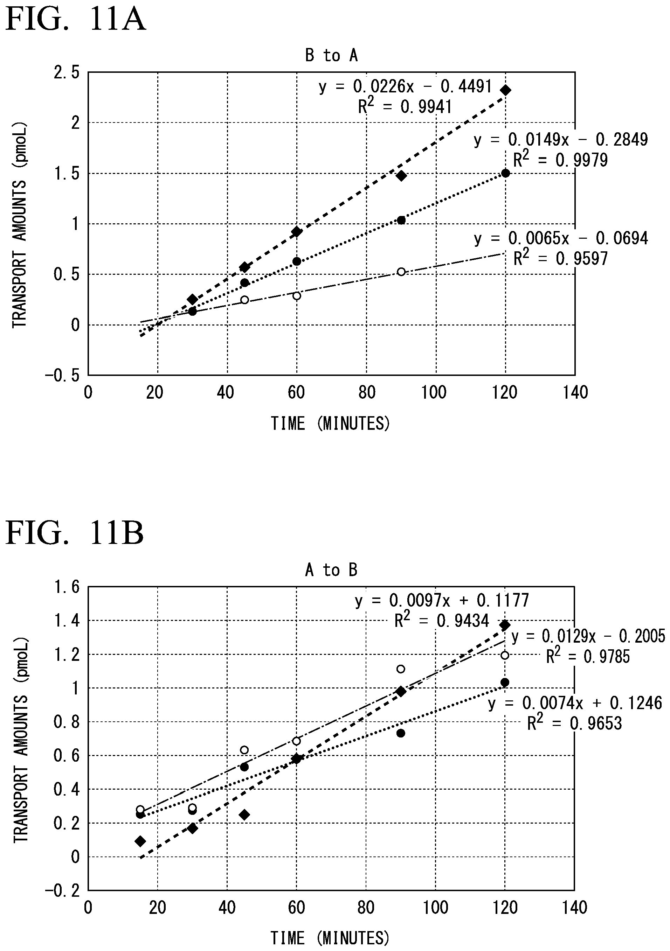

FIG. 11A is a graph showing the results of measuring the transport amount (BtoA) three times, and FIG. 11B is a graph showing the results of measuring the transport amount (AtoB) three times. The axis of abscissa in the graph represents the time (minutes), and the axis of ordinate represents the amount (pmoL) of transported CDCFDA. The efflux ratio calculated in the same manner as in Experimental Example 3 from the average value of the results of the three measurements was about 1.5.

[Experimental Example 10]

(Evaluation of BCRP)

<<Measurement of efflux ratio by Hoechst 33342>>

The efflux ratio of Hoechst 33342 (CAS number: 23491-52-3), which is a substrate for BCRP, was measured by using the biomimetic system.

Human cholangiocyte-like cells were manufactured by a method similar to that used in Experimental Example 9. In addition, a biomimetic system was manufactured in the same manner as in Manufacturing Example 1 of Experimental Example 2, except that the number of culture days was set to 15 days. The TEER of the manufactured biomimetic system was measured by the same method as that used in Experimental Example 2, and the value was 284.83 $\Omega \cdot cm^2$.

The transport amounts of Hoechst 33342 were measured in the same manner as in Experimental Example 3, except that Hoechst 33342 was used in place of rhodamine 123 as a substrate. Regarding Hoechst 33342, a solution obtained by dissolving the substance in deionized water to a concentration of 1% by mass was used.

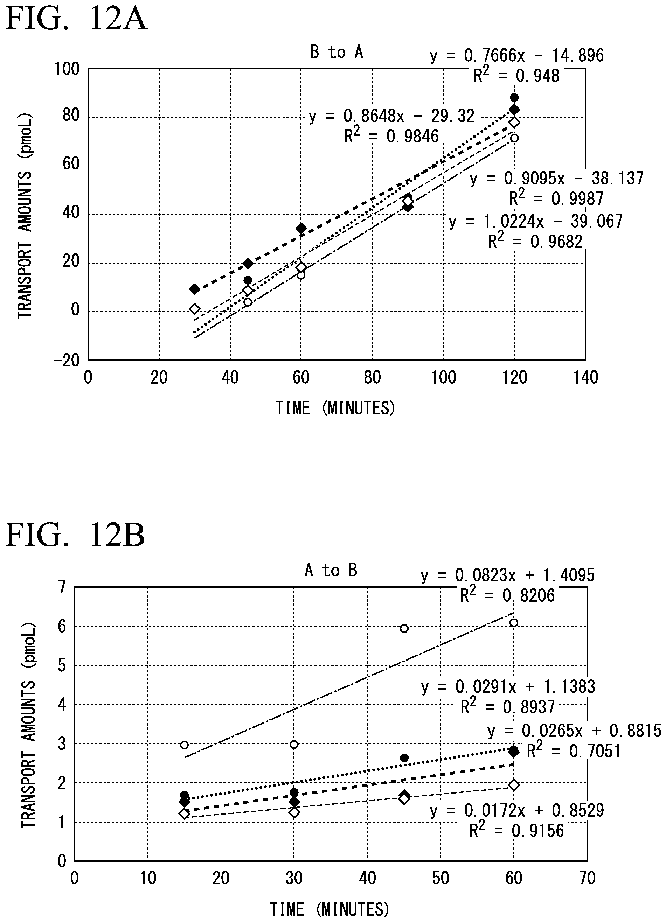

FIG. 12A is a graph showing the results of measuring the transport amount (BtoA) four times, and FIG. 12B is a graph showing the results of measuring the transport amount (AtoB) four times. The axis of abscissa in the graph represents the time (minutes), and the axis of ordinate represents the amount (pmoL) of transported Hoechst 33342. The efflux ratio calculated in the same manner as in Experimental Example 3 from the average value of the results of the four measurements was about 23.0.

«Verification of effect of BCRP inhibitor»

The effect of Ko143 (CAS Number: 461054-93-3), which is a BCRP inhibitor, was checked.

The transport amounts of Hoechst33342 were measured in the same manner as in Experimental Example 5, except that Hoechst 33342 was used in place of rhodamine 123, Ko143 was used in place of verapamil hydrochloride, and Hoechst 33342 in an amount to reach 10 $\mu$M and Ko143 in an amount to reach 20 $\mu$M were added to the culture medium on the side A. Regarding Hoechst 33342 and Ko143, a solution obtained by dissolving each of them in deionized water to a concentration of 0.1% by mass was used.

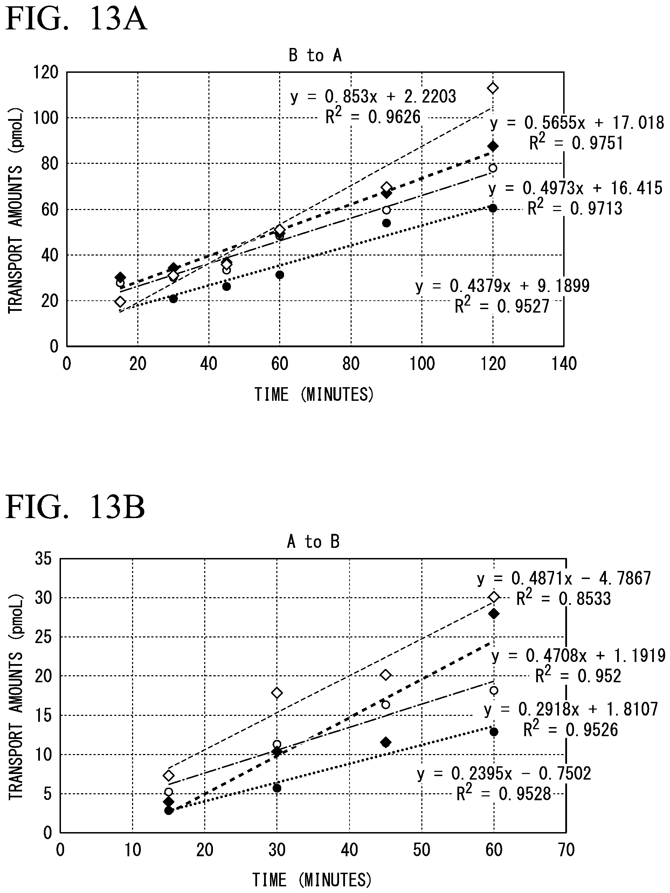

FIG. 13A is a graph showing the results of measuring the transport amount (BtoA) four times, and FIG. 13B is a graph showing the results of measuring the transport amount (AtoB) four times. The axis of abscissa in the graph represents the time (minutes), and the axis of ordinate represents the amount (pmoL) of transported Hoechst 33342. The efflux ratio calculated in the same manner as in Experimental Example 3 from the average value of the results of the four measurements was about 1.6.

[Industrial Applicability]

According to the present invention, a biomimetic system useful for the evaluation of biliary excretion, and a method for manufacturing the biomimetic system can be provided.

[Reference Signs List]

100: Biomimetic system
10: Container
20: Human cholangiocyte-like cell-containing membrane
30: Permeable base plate
31: Surface
40: Human cholangiocyte-like cell
50: First compartment
60: Second compartment

**Claims**

1.   A biomimetic system comprising:

a container; and
a human cholangiocyte-like cell-containing membrane,
wherein the human cholangiocyte-like cell-containing membrane includes a permeable base plate and a two-dimensional tissue of human cholangiocyte-like cells stacked on one surface of the permeable base plate,
the human cholangiocyte-like cell-containing membrane divides the container into a first compartment and a second compartment,
the one surface side of the permeable base plate is exposed in the first compartment,
the other surface side of the permeable base plate is exposed in the second compartment,
the human cholangiocyte-like cells express P-glycoprotein (P-gp), and
an efflux ratio calculated by Formula (1) is 1.5 or more,

Efflux ratio = (Permeation rate of rhodamine 123 permeating from the second compartment to the first compartment in a case where rhodamine 123 (CAS number: 62669-70-9) is added to the second compartment)/(permeation rate of rhodamine 123 permeating from the first compartment to the second compartment in a case where rhodamine 123 is added to the first compartment)          (1)

2.   The biomimetic system according to Claim 1,
wherein a transepithelial electrical resistance of the two-dimensional tissue is 100 $\Omega \cdot cm^2$ or greater.

3.   The biomimetic system according to Claim 1,
wherein the two-dimensional tissue is a single layer of the human cholangiocyte-like cells.

4.   The biomimetic system according to Claim 1,
wherein the permeable base plate is a base plate coated with collagen or laminin, or a combination of collagen and laminin.

5.   The biomimetic system according to Claim 1,
wherein the human cholangiocyte-like cells further express at least one or more transporters selected from the group consisting of MRP2, BSEP, and BCRP.

6. A method for manufacturing a biomimetic system, the method comprising:

   a step of seeding human cholangiocyte-like cells on one surface of a permeable base plate in a container divided into a first compartment and a second compartment by the permeable base plate; and
   a step of culturing the human cholangiocyte-like cells in a culture medium including 0.001 to 5 v/v% of an extracellular matrix so that, consequently, a human cholangiocyte-like cell-containing membrane in which a two-dimensional tissue of the human cholangiocyte-like cells is stacked on the one surface of the permeable base plate is formed.

7. The method for manufacturing a biomimetic system according to Claim 6,
   wherein the extracellular matrix includes laminin.

8. A method for manufacturing a biomimetic system, the method comprising:

   a step of seeding human cholangiocyte-like cells on one surface of a permeable base plate of a container divided into a first compartment and a second compartment by the permeable base plate; and
   a step of culturing the human cholangiocyte-like cells in a culture medium so that, consequently, a human cholangiocyte-like cell-containing membrane in which a two-dimensional tissue of the human cholangiocyte-like cells is stacked on the one surface of the permeable base plate is formed,
   wherein the permeable base plate is a base plate coated with collagen or laminin, or a combination of collagen and laminin.

9. The method for manufacturing a biomimetic system according to Claim 6 or 8,
   wherein a seeding density of the human cholangiocyte-like cells is 5,000 to 200,000 cells/cm$^2$.

10. The method for manufacturing a biomimetic system according to Claim 6 or 8,
    wherein the culture medium contains one kind or two or more kinds of factors selected from the group consisting of a Wnt signaling pathway activator, epidermal growth factor (EGF), a fibroblast growth factor (FGF), hepatocyte growth factor (HGF), and a cAMP signaling pathway activator.

11. The method for manufacturing a biomimetic system according to Claim 6 or 8,
    wherein the culture medium contains a TGFβ signaling pathway inhibitor.

12. The method for manufacturing a biomimetic system according to Claim 6 or 8,
    wherein the culture medium substantially does not contain one kind or two or more kinds selected from the group consisting of DAPT, a Wnt signaling pathway inhibitor, and dimethyl sulfoxide (DMSO).

13. The method for manufacturing a biomimetic system according to Claim 6 or 8,
    wherein the human cholangiocyte-like cells are a human cholangiocyte-like cell line established from human primary hepatocytes.

14. A biomimetic system obtained by the method for manufacturing a biomimetic system according to Claim 6 or 8.

15. A method for screening a test substance, the method comprising:
    a step of bringing a test substance into contact with the human cholangiocyte-like cell-containing membrane of the biomimetic system according to any one of Claims 1 to 5.

FIG. 1

# FIG. 2

# FIG. 3

MANUFACTURING EXAMPLE 1    MANUFACTURING EXAMPLE 2

# FIG. 4A

DAY 17

y=1.6948x-20.608
$R^2$=0.9287

y=0.8562x+19.599
$R^2$=0.9684

Legend: ⊛ A to B, ○ B to A

Y-axis: TRANSPORT AMOUNTS (pmoL)

X-axis: TIME (MINUTES)

# FIG. 4B

DAY 23

y=0.67x-6.0728
$R^2$=0.9463

y=0.2984x+2.7504
$R^2$=0.9818

Legend: ⊛ A to B, ○ B to A

Y-axis: TRANSPORT AMOUNTS (pmoL)

X-axis: TIME (MINUTES)

FIG. 5

MANUFACTURING EXAMPLE 3

200μm

MANUFACTURING EXAMPLE 4

200μm

MANUFACTURING EXAMPLE 5

200μm

MANUFACTURING EXAMPLE 6

200μm

MANUFACTURING EXAMPLE 7

200μm

MANUFACTURING EXAMPLE 8

200μm

FIG. 6A

0 v/v%

FIG. 6B

0.1 v/v%

FIG. 6C

0.5 v/v%

## FIG. 6D

2 v/v%

y=0. 2666x−1. 397
R²=0. 8711

y=0. 2046x+0. 8377
R²=0. 9173

- ⊛ A to B
- ○ B to A

## FIG. 7A

2 v/v%

y=0. 1776x−0. 5945
R²=0. 9997

y=0. 036x+2. 0839
R²=0. 951

- ⊛ A to B
- ○ B to A

## FIG. 7B

5 v/v%

y=0. 236x−1. 957
R²=0. 9612

y=0. 0502x+1. 583
R²=0. 9672

- ⊛ A to B
- ○ B to A

## FIG. 7C

10 v/v%

A to B
B to A

y=0.1929x−0.5465
$R^2$=0.9973

y=0.0496x+1.8652
$R^2$=0.997

TRANSPORT AMOUNTS (pmoL)

TIME (MINUTES)

EP 4 421 163 A1

FIG. 8

FIG. 9

FIG. 10A

FIG. 10B

## FIG. 11A

B to A

$y = 0.0226x - 0.4491$
$R^2 = 0.9941$

$y = 0.0149x - 0.2849$
$R^2 = 0.9979$

$y = 0.0065x - 0.0694$
$R^2 = 0.9597$

TRANSPORT AMOUNTS (pmoL)

TIME (MINUTES)

## FIG. 11B

A to B

$y = 0.0097x + 0.1177$
$R^2 = 0.9434$

$y = 0.0129x - 0.2005$
$R^2 = 0.9785$

$y = 0.0074x + 0.1246$
$R^2 = 0.9653$

TRANSPORT AMOUNTS (pmoL)

TIME (MINUTES)

FIG. 12A

FIG. 12B

## FIG. 13A

B to A

$y = 0.853x + 2.2203$
$R^2 = 0.9626$

$y = 0.5655x + 17.018$
$R^2 = 0.9751$

$y = 0.4973x + 16.415$
$R^2 = 0.9713$

$y = 0.4379x + 9.1899$
$R^2 = 0.9527$

TRANSPORT AMOUNTS (pmoL)

TIME (MINUTES)

## FIG. 13B

A to B

$y = 0.4871x - 4.7867$
$R^2 = 0.8533$

$y = 0.4708x + 1.1919$
$R^2 = 0.952$

$y = 0.2918x + 1.8107$
$R^2 = 0.9526$

$y = 0.2395x - 0.7502$
$R^2 = 0.9528$

TRANSPORT AMOUNTS (pmoL)

TIME (MINUTES)

<div align="center">

**INTERNATIONAL SEARCH REPORT**

</div>

| International application No. |
|---|
| **PCT/JP2022/038703** |

**A.    CLASSIFICATION OF SUBJECT MATTER**

*C12N 5/00*(2006.01)i; *C12N 5/071*(2010.01)i; *C12Q 1/02*(2006.01)i; *G01N 33/15*(2006.01)i
FI:    C12N5/071; C12Q1/02; C12N5/00; G01N33/15 Z

According to International Patent Classification (IPC) or to both national classification and IPC

**B.    FIELDS SEARCHED**

Minimum documentation searched (classification system followed by classification symbols)

C12N5/00; C12N5/071; C12Q1/02; G01N33/15

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

JSTPlus/JMEDPlus/JST7580 (JDreamIII); CAplus/MEDLINE/EMBASE/BIOSIS (STN)

**C.    DOCUMENTS CONSIDERED TO BE RELEVANT**

| Category* | Citation of document, with indication, where appropriate, of the relevant passages | Relevant to claim No. |
|---|---|---|
| Y | WO 2021/125176 A1 (JSR CORPORATION) 24 June 2021 (2021-06-24) claims, examples, paragraphs [0110]-[0112] | 1-5, 8-15 |
| A | entire text | 6-7 |
| Y | ASAI, Akihiro et al. Paracrine signals regulate human liver organoid maturation from induced pluripotent stem cells. DEVELOPMENT. 2017, vol. 144, pp. 1056-1064 abstract, p. 1058, left column, second paragraph, p. 1063, left column, third paragraph, fig. 3 | 1-5, 8-15 |
| A | entire text | 6-7 |
| Y | JP 2021-126073 A (NATIONAL AGRICULTURE AND FOOD RESEARCH ORGANIZATION) 02 September 2021 (2021-09-02) claims, examples, fig. 5 | 1-5, 8-15 |
| A | entire text | 6-7 |

☑ Further documents are listed in the continuation of Box C.      ☑ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **16 November 2022** | **06 December 2022** |

| Name and mailing address of the ISA/JP | Authorized officer |
|---|---|
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (January 2015)

**INTERNATIONAL SEARCH REPORT**

| International application No. |
| :--- |
| **PCT/JP2022/038703** |

**C.    DOCUMENTS CONSIDERED TO BE RELEVANT**

| Category* | Citation of document, with indication, where appropriate, of the relevant passages | Relevant to claim No. |
| :---: | :--- | :---: |
| Y | TANG, Fuxing et al. Bidirectional Transport of Rhodamine 123 and Hoechst 33342, Fluorescence Probes of the Binding Sites on P-glycoprotein, across MDCK-MDR1 Cell Monolayers. Journal of Pharmaceutical Sciences. 2004, vol. 93, no. 5, pp. 1185-1194 abstract, p. 1187, left column, second paragraph, table 1 | 1-5, 8-15 |
| A | entire text | 6-7 |

Form PCT/ISA/210 (second sheet) (January 2015)

## INTERNATIONAL SEARCH REPORT
### Information on patent family members

International application No.

**PCT/JP2022/038703**

| Patent document cited in search report | | | Publication date (day/month/year) | Patent family member(s) | Publication date (day/month/year) |
|---|---|---|---|---|---|
| WO | 2021/125176 | A1 | 24 June 2021 | (Family: none) | |
| JP | 2021-126073 | A | 02 September 2021 | (Family: none) | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 2021170989 A

### Non-patent literature cited in the description

- **CHEN C. et al.** Kanglaite enhances the efficacy of cisplatin in suppression of hepatocellular carcinoma via inhibiting CKLF1 mediated NF-kappaB pathway and regulating transporter mediated drug efflux. *Journal of Ethnopharmacology,* 2021, vol. 264, 113388
- **ASAI A. et al.** Paracrine signals regulate human liver organoid maturation from induced pluripotent stem cells. *Development,* 2017, vol. 144, 1056-1064
- **TAKAYAMA K. et al.** Prediction of interindividual differences in hepatic functions and drug sensitivity by using human iPS-derived hepatocytes. *PNAS,* 2014, vol. 111 (47), 16772-16777
- *CHEMICAL ABSTRACTS,* 252917-06-9
- *CHEMICAL ABSTRACTS,* 280744-09-4
- *CHEMICAL ABSTRACTS,* 264218-23-7
- *CHEMICAL ABSTRACTS,* 252935-94-7
- *CHEMICAL ABSTRACTS,* 612487-72-6
- *CHEMICAL ABSTRACTS,* 603288-22-8
- *CHEMICAL ABSTRACTS,* 66575-29-9
- *CHEMICAL ABSTRACTS,* 16980-89-5
- *CHEMICAL ABSTRACTS,* 9012-63-9
- *CHEMICAL ABSTRACTS,* 317-34-0
- *CHEMICAL ABSTRACTS,* 51-28-5
- *CHEMICAL ABSTRACTS,* 108341-18-0
- *CHEMICAL ABSTRACTS,* 51-43-4
- *CHEMICAL ABSTRACTS,* 7683-59-2
- *CHEMICAL ABSTRACTS,* 28822-58-4
- *CHEMICAL ABSTRACTS,* 58-08-2
- *CHEMICAL ABSTRACTS,* 58-55 -9
- *CHEMICAL ABSTRACTS,* 61413-54-5
- *CHEMICAL ABSTRACTS,* 73873-87-7
- *CHEMICAL ABSTRACTS,* 745-65-3
- *CHEMICAL ABSTRACTS,* 909910-43-6
- *CHEMICAL ABSTRACTS,* 301836-41-9
- *CHEMICAL ABSTRACTS,* 694433-59-5
- *CHEMICAL ABSTRACTS,* 356559-20-1
- *CHEMICAL ABSTRACTS,* 396129-53-6
- *CHEMICAL ABSTRACTS,* 627536-09-8
- *CHEMICAL ABSTRACTS,* 446859-33-2
- *CHEMICAL ABSTRACTS,* 208255-80-5
- *CHEMICAL ABSTRACTS,* 62669-70-9
- *CHEMICAL ABSTRACTS,* 127770-45-0
- *CHEMICAL ABSTRACTS,* 115104-28-4
- *CHEMICAL ABSTRACTS,* 23491-52-3
- *CHEMICAL ABSTRACTS,* 461054-93-3